# EUROPEAN PATENT APPLICATION

(11) **EP 2 394 668 A1**
(43) Date of publication of application: **14.12.2011**
(21) Application number: 10164663.6
(22) Date of filing: 01.06.2010
(51) Int. Cl.: A61K 51/04

(54) **PET radiopharmaceuticals for differential diagnosis between bilateral and unilateral conditions of primary hyperaldosteronism**

(71) Applicant: Julius-Maximilians-Universität Würzburg, 97970 Würzburg (DE)
(72) Inventor: The designation of the inventor has not yet been filed
(74) Representative: Lelkes, Robert

(57) **Abstract**

A functional PET imaging method is disclosed for differentiation between bilateral hyperplasia and unilateral adenoma comprising (1) introducing a radioactively labelled CYP11 B2 (aldosterone synthase) inhibitor which binds selectively to CYP11 B2 (aldosterone synthase) relative to CYP11 B1 (11β-hydroxylase) into a mammal with adrenal glands and (2) conducting positron emission tomography (PET) in the region of the adrenal glands to obtain a functional PET image of the adrenal glands. Also disclosed are radioactive tracer compounds suitable for use in this method, precursors for making the same, and a process for making the radioactive tracer compounds capable of being conducted as a rapid one-pot reaction.

## Description

### Background of the Invention

Primary hyperaldosteronism poses difficult diagnostic challenges. Recent studies have shown that this disease of the adrenal glands, with a prevalence of 5-12% of all hypertensive patients, represents the most common monocausal factor in arterial hypertension. Assuming that 25-30% of the population suffers from arterial hypertension, and that primary hyperaldosteronism is present in 5% of the cases, this results in a calculated prevalence of 1250-1500 cases per 100,000 persons.

The key diagnostic problem in confirmed primary hyperaldosteronism is differentiation between bilateral hyperplasia and unilateral adenoma (Conn adenoma). This classification is critical for further treatment. While primary hyperaldosteronism based on a unilateral condition (Conn adenoma) may be successfully treated by surgery, bilateral hyperplasia is treated conservatively by medication.

Due to the high prevalence of endocrine-inactive adrenal gland incidentalomas, which increases with age, conventional imaging has only limited suitability for this differentiation. In addition, it is frequently not possible to reliably image unilateral Conn adenomas of small size. Therefore, the current "gold standard" for further diagnostics is the bilateral sampling and analysis of blood from the adrenal veins.

Blood sampling from both adrenal veins, considered to be the gold standard, is an invasive procedure with associated side effects. This method therefore requires an experienced clinician, and has become established at only a few medical centers. However, due to the great technical difficulties, complications (e.g. adrenal hemorrhage) occur in just under 20% of adrenal venous catheters, and in a high percentage of tests (approximately 40%) selective cannulation of one or both adrenal veins fails and no usable results are obtained. Thus, the gold standard is greatly in need of improvement.

The informative value of magnetic resonance tomography (MRT) or computed tomography (CT) is very limited, especially for patients over 40 years of age: on the one hand, with increasing life expectancy more hormonally inactive adrenal masses are detected, and on the other hand Conn adenomas may be very small (< 5 mm) and thus escape detection by imaging.

The present invention overcomes the shortcomings of the above-described state of the art.

### Summary of the Invention

According to the present invention, a functional imaging method is provided for differentiation between bilateral hyperplasia and unilateral adenoma comprising (1) introducing a radioactively labelled CYP11 B2 (aldosterone synthase) inhibitor which binds selectively to CYP11 B2 (aldosterone synthase) relative to CYP11 B1 (11β-hydroxylase) into a mammal with adrenal glands and (2) conducting positron emission tomography (PET) in the region of the adrenal glands to obtain a functional image of the adrenal glands.

The present invention also relates to compounds that may be used as radioactively labeled CYP11 B2 inhibitors in the above method, or their precursors, having the formula (I): 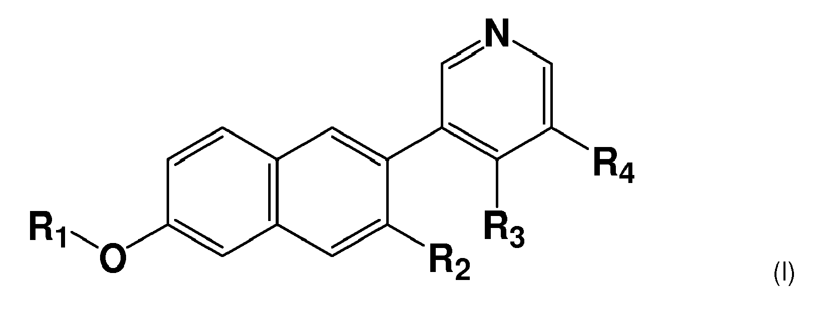 wherein
R₁ represents ―(CH₂)₂-X or ―CH₃;
R₂ represents ―H, -CH₂O(CH₂)₂-X or ―C(O)O(CH₂)₂-X;
R₃ represents ―H, -CH₃, -C(O)-N-pyrrolidine, -CH₂-N-pyrrolidine, or ―N- pyrrolidine
R₄ represents ―H, -CH₂-O-CH₃, -CH(CH₃)OCH₃, -C(O)-N-pyrrolidine, -CH₂-N-pyrrolidine, -N-pyrrolidine, -CH(CH₃)-X, -O(CH₂)₂-X, -O(CH₂)₃-X, -CH₂-O-(CH₂)₂-X, or ―CH(CH₃)-O-(CH₂)₂-X, or
R₁ and R₂ are defined as above and R₃ and R₄ together with the pyridinyl ring of formula (I) form a isochinoline ring system,
wherein one of R₁, R₂ or R₄ represents a group having an X moiety and X represents ¹⁸F, Br, I, tosylate or mesylate.

When the compound is intended for use as a radioactive tracer, X represents ¹⁸F. When the compound is intended for use as a precursor for making a tracer, X represents Br, I, tosylate or mesylate.

The present invention also includes a process for making a radioactive tracer comprising reacting a compound of formula (I), wherein R₁ to R₄ are defined as above, one of R₁, R₂ or R₄ represents a group having an X moiety and X represents Br, I, tosylate or mesylate, with ¹⁸F ions, preferably in the presence of a catalyst, to obtain the radioactive tracer of the present invention.

### Detailed Description of the Invention

The enzyme aldosterone synthase (CYP11B2) is specifically expressed in the aldosterone-producing tissue (zona glomerulosa) of the adrenal glands. Expression of this enzyme at levels up to 10 times higher has been documented in Conn adenomas as well as in bilateral hyperplasias. The functional imaging method of the present invention is able to represent the activity of aldosterone synthase, thereby allowing differentiation between the two main forms of hyperaldosteronism. In unilateral hyperplasia, the contralateral side is suppressed, and therefore in contrast to bilateral hyperplasia a clear difference between sides is detectable when subjected to positron emission tomography (PET) scanning.

The PET imaging method allows absolute quantification of tracer concentrations. In a preferred embodiment, PET imaging is conducted with a PET/CT or PET/MRT device, which allow unequivocal anatomical assignment of a tracer enrichment observed using PET.

The radioactive tracer comprises at least one radioactive isotope. The radioactive isotope is preferably an isotope of a halogen. More preferably, the radioactive isotope is a fluorine isotope have an atomic weight of 18, referred to as ¹⁸F. The PET nuclide ¹⁸F, with a physical half-life of 110 minutes, may be routinely produced in very high activity levels on any cyclotron. The ¹⁸F may be isolated from the ¹⁸O⁻ water coming out of the cyclotron target by using a quaternary ammonium anion exchange column. The retained ¹⁸F⁻ is eluted with a solution comprising a cryptand, such as Kryptofix™222, a cyclic crown ether available from Merck, cat. No. 814925, CAS-No. 23978-09-8, an appropriate potassium salt, such as potassium carbonate, in a polar aprotic organic solvent such as acetonitrile.

The fluorine isotope is thereby available as [¹⁸F] KF in the presence of the aforementioned eluent solution. The mixture is preferably evaporated to dryness at an elevated temperature, such as 85°C, in the presence of an inert gas, such as argon, to form a residue, which is then azeotropically dried in the presence of an anhydrous polar aprotic organic solvent, such as acetonitrile, in the presence of an inert gas, such as argon. The reaction is conducted by adding the precursor to the dehydrated product.

The reaction between [¹⁸F] KF and the precursors of the present invention is preferably conducted in the presence of the aforementioned cryptand in a solvent comprising a polar aprotic organic solvent, such as acetonitrile or another polar aprotic solvent such as N,N-dimethyl formamide (DMF) or dimethyl sulfoxide (DMSO).

The reaction is preferably carried out at a temperature of at least 70°C, more preferably at least 80°C up to 180°C, more preferably up to 100°C, even more preferably up to 90°C. In particular, the reaction is preferably carried out at about 80°C (e.g., +/- 5°C) to minimize decomposition processes.

Due to the relative short half-life time of F-18, the reaction is preferably conducted for a minimal time period, preferably less than 20 minutes. The reaction is conducted under conditions of time, temperature and concentration effective for obtaining a desired radioactive yield. Such conditions are easily established via routine experimentation and/or common general knowledge in the field of chemistry.

The radioactive tracer is then isolated from the other components of the reaction mixture. This step may, for example, be conducted via a chromatographic method, such as high pressure liquid chromatography (HPLC). For example, after cooling the reaction mixture to room temperature, the solution containing the reaction product may be loaded directly onto an HPLC column containing Kromasil 100-10 C18 and eluted at 7 mL/min with the relative amounts of CH₃OH/H₂O/triethylamine at 70:30:0.1 by volume (v/v/v).

The radiochemical yield is preferably at least 10 percent, more preferably at least 15 percent.

The precursors may be synthesized by reacting a compound having a hydroxy group in the X position of formula (I) by standard bromination reactions known in the literature, e.g. with tetrabromomethane in the presence of triphenylphosphine.

Compounds having an hydroxy group in the X-position of R₁ may be made by reacting a pyridine compound having Br or I in the 3-position and ―H, -CH₂-O-CH₃, -CH(CH₃)OCH₃, -C(O)-N-pyrrolidine, -CH₂-N-pyrrolidine, ―N-pyrrolidine in the 5-position with 6-hydroxyethoxy-2-naphthalene boronic acid.

Compounds having an hydroxy group in the X-position of R₂ may be made by reacting a pyridine compound having Br or I in the 3-position and ―H, -CH₂-O-CH₃, -CH(CH₃)OCH₃, -C(O)-N-pyrrolidine, -CH₂-N-pyrrolidine, -N-pyrrolidine in the 5-position with 6-methoxy-2-naphthalene boronic acid having a -CH₂O(CH₂)₂-OH or ―C(O)O(CH₂)₂-OH substituent in the 3-position.

Compounds having an hydroxy group in the X-position of R₄ may be made by reacting a pyridine compound having Br or I in the 3-position and a hydroxy group in the 5-position with 6-methoxy-2-naphthalene boronic acid.

The last method is illustrated by the following synthesis example.

### SYNTHESIS EXAMPLE 1

### Step 1: Synthesis of 3-bromo-5-(2-hydroxyethoxy)pyridine

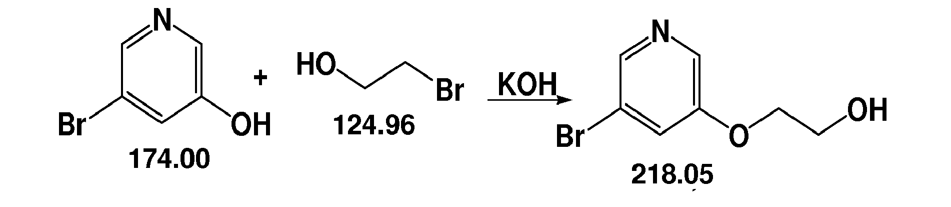

To a solution of 2.0 g (11.5 mmol) 3-bromo-5-hydroxypyridine (glassware evacuated) and 1.0 mL (1.75 g, 14 mmol) 2-bromoethanol in 10 mL DMF was added a solution of 920 mg (13.9 mmol) potassium hydroxide and 63 mg (0.37 mmol) potassium iodide in 2 mL water, and the mixture was heated at 85°C for 4 h. After cooling to room temperature the mixture was filtered, and the filtrate was diluted with 70 mL water and 70 mL diethyl ether. The organic phase was separated and washed with 20 mL 2% aqueous KOH solution. After stripping the solvent, the crude product was obtained as a light-colored solid (melting point: 53― 55°C).

Product characteristics:
Yield: 339 mg (1.55 mmol, 13.5%)

Thin-layer chromatography (silica gel):
R_{f} 3-bromo-5-hydroxypyridine (CH₂Cl₂) = 0.05
R_{f} 3-bromo-5-(2-hydroxyethoxy)pyridine (CH₂Cl₂) = 0.05
¹H-NMR (CDCl₃): δ = 8.30 (d, 1H), 8.25 (d, 1H), 7.40 (t, 1H), 4.15 (m, 2H), 4.00 (m, 2H), 2.35 (bs, 1H)

### Step 2: 3-(6-Methoxy-2-naphthyl)-5-(2-hydroxyethoxy)pyridine

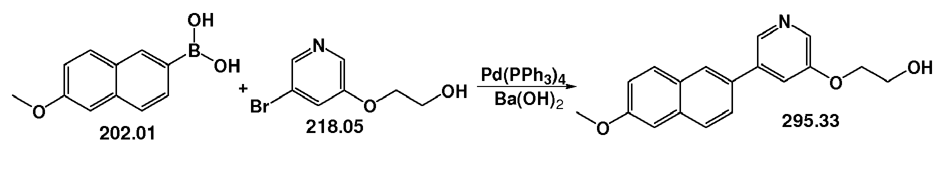

A flask containing 375 mg (1.85 mmol) 6-methoxy-2-naphthalene boronic acid, 339 mg (1.55 mmol) 3-bromo-5-(2-hydroxyethoxy)pyridine, 729 mg (2.32 mmol) barium hydroxide octahydrate, and 72 mg (0.06 mmol) tetrakis(triphenylphosphine)palladium was flushed with argon for 5 min. 9 mL dimethoxyethane and 1.5 mL water were then added by injecting through a septum via syringe, and the mixture was heated overnight at 80°C, under argon. After stripping the solvent, the residue was dissolved in 50 mL water, extracted three times each with 25 mL chloroform, and dried over sodium sulfate. After stripping the solvent the crude product was purified by column chromatography (CH₂Cl₂/MeOH 95/5).

Purified product characteristics:
Appearance: White solid
Melting point: 153-156°C
Yield: 248 mg (0.84 mmol, 54.2%)

Thin-layer chromatography (silica gel):
R_{f} 3-bromo-5-(2-hydroxyethoxy)pyridine (CH₂Cl₂/CH₃OH 95/5)= 0.30
R_{f} 3-(6-methoxy-2-naphthyl)-5-(2-hydroxyethoxy)pyridine (CH₂Cl₂/CH₃OH 95/5) = 0.25
¹H-NMR (CDCl₃): δ = 8.60 (bs, 1H), 8.30 (bs, 1H), 7.95 (s, 1H), 7.80 (t, 2H), 7.65 (d, 1H), 7.50 (m, 1H), 7.15 (m, 2H), 4.25 (t, 2H), 4.05 (t, 2H), 3.95 (s, 3H), 2.40 (bs, 1H)

### Step 3: 3-(6-Methoxy-2-naphthyl)-5-(2-bromoethoxy)pyridine (precursor)

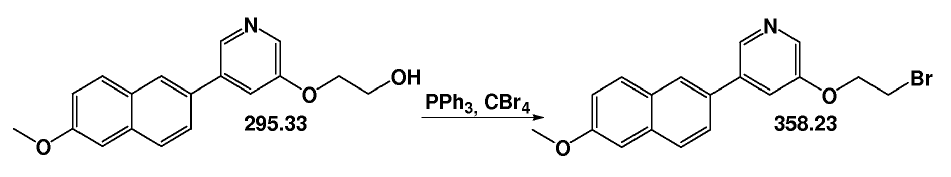

A solution of 248 mg (0.84 mmol) 3-(6-methoxy-2-naphthyl)-5-(2-hydroxyethoxy)pyridine and 441 mg (1.68 mmol) triphenylphosphine in 7 mL dichloromethane was cooled in an ice bath, 332 mg (1.0 mmol) tetrabromomethane was added, and stirring was performed in the ice bath for 1 h. After stripping the solvent the crude product was purified by column chromatography (CH₂Cl₂/MeOH 97/3).

Purified product characteristics:
Appearance: Yellow crystals
Melting point: 106-110°C
Yield: 238 mg (0.80 mmol, 95.9%)

Thin-layer chromatography (silica gel):
R_{f} 3-(6-methoxy-2-naphthyl)-5-(2-hydroxyethoxy)pyridine (CH₂Cl₂/CH₃OH 95/5) = 0.25
R_{f} 3-(6-methoxy-2-naphthyl)-5-(2-bromoethoxy)pyridine (CH₂Cl₂/CH₃OH 95/5) = 0.40
¹H-NMR (CDCl₃): δ = 8.65 (bs, 1H), 8.30 (bs, 1H), 7.95 (s, 1H), 7.75 (t, 2H), 7.70 (d, 1H), 7.50 (m, 1H), 7.20 (m, 2H), 4.45 (t, 2H), 3.95 (s, 3H), 3.70 (t, 2H)

### Radiosynthesis of [¹⁸F] 3-(6-methoxy-2-naphthyl)-5-(2-fluoroethoxy)pyridine:

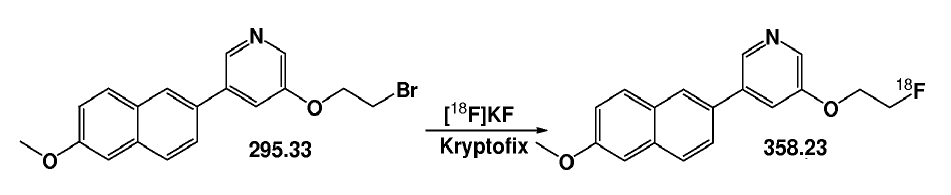

From a cartridge in which [¹⁸F] potassium fluoride produced in a cyclotron had been fixed, the radionuclide was eluted with a solution composed of 900 µL acetonitrile, 100 µL water, 20 mg Kryptofix, and 30 µL 1 M K₂CO₃ solution, and the mixture was evaporated to dryness at 85°C under an argon stream. The residue was then azeotropically dried two times each with 1 mL anhydrous acetonitrile under an argon stream. A solution of 5 mg of 3-(6-methoxy-2-naphthyl)-5-(2-bromoethoxy)pyridine was then added, and the mixture was heated at 120°C for 20 min. After cooling to room temperature the solution was loaded directly onto the HPLC (Kromasil 100-10 C18, 7 mL/min CH₃OH/H₂O/triethylamine 70/30/0.1 v/v/v). The radiochemical yield was 20%.

### SYNTHESIS EXAMPLE 2

### Step 1: 2-Bromo-6-(2-fluoroethoxy)naphthalene

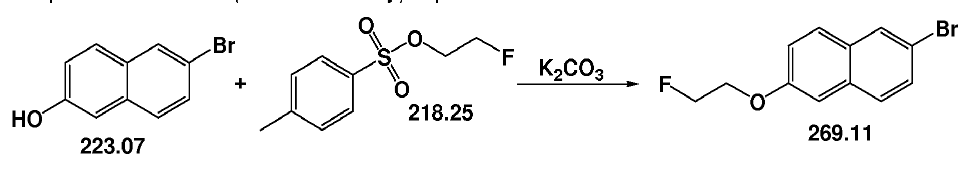

A solution of 2.37 g (10.6 mmol) 2-Bromo-6-naphthol, 3.42 g (24.4 mmol) K₂CO₃ and 4.16 g (19.1 mmol) 2-Fluoroethyltosylate in 25 mL DMF was heated overnight at 60°C. The solution was poured into 300 mL water and extracted with chloroform (3 x 100 mL). The organic phases were washed with 100 mL 1 N NaOH and 100 ml water and dried over Na₂SO₄. After stripping the solvent the crude product was purified by column chromatography (Hexane/EtOAc 80/20).

Product characteristics:
Appearance: Yellow solid
Melting point: 83-85°C
Yield: 2.70 g (10.0 mmol, 94.3%)

Thin-layer chromatography (silica gel):
R_{f} 2-Bromo-6-naphthol (Hexane/EtOAc 80/20) = 0.45
R_{f} 2-Bromo-6-(2-fluoroethoxy)naphthalene (Hexane/EtOAc 80/20) = 0.65
¹H-NMR (CDl₃): δ = 7.80-7.10 (m, 6H), 4.90 (t, 1H), 4.75 (t, 1H), 4.30 (t, 1H), 7.15 (t, 1H).

### Step 2: 2-(2-Fluoroethoxy)naphthalene-6-boronic acid

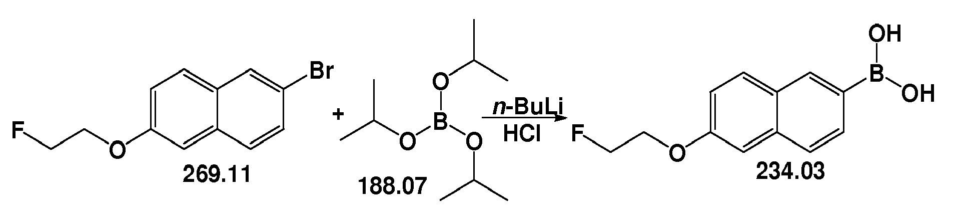

A solution of 3.5 mL (2.84 g, 15.1 mmol) Boronic acid triisopropylester and 3.35 g (12.4 mmol) 2-Bromo-6-(2-fluoroethoxy)naphthalene in 19 mL toluene and 5 mL THF was cooled under argon to -40°C. 6.3 ml (15.7 mmol) of a 2.5 M n-Butyllithium-solution in hexane was added via a syringe. After 30 min the solution was warmed to -20°C and 13 ml 2 N HCl were added dropwise. The aqueous phase was separated, neutralized by addition of solid NaOH and saturated with 5 g NaCl. After extraction with THF (3 x 30 mL) the solvent was stripped under reduced pressure and the crude product heated at 70°C with 13 mL acetonitrile. After cooling in the refrigerator the product precipitated.

Product characteristics:
Appearance: White solid
Melting point: > 200°C
Yield: 1.96 g (8.4 mmol, 67.5%)
Insoluble in organic solvents; therefore no ¹H-NMR-characterization

### Step 3: 3-(6-(2-Fluoroethoxy)-2-naphthyl)-4-methylpyridine

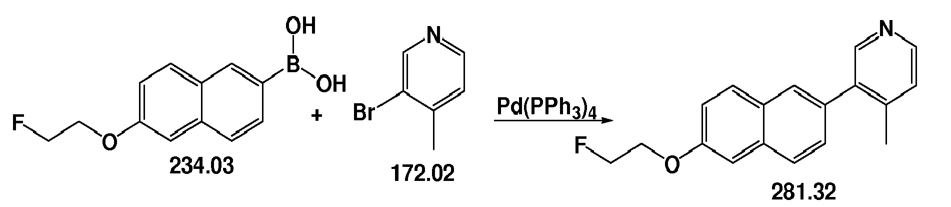

A solution of 111 µL (172 mg, 1.0 mmol) 3-Bromo-4-methylpyridine, 44 mg (0.04 mmol) Tetrakis triphenylphosphine palladium(0), 281 mg (1.2 mmol) 2-(2-Fluoroethoxy)-2-naphthalene-6-boronic acid and 474 mg (1.51 mmol) Bariumhydroxid Octahydrate was purged with argon. 6 mL Dimethoxyethane and 1 ml water were added via a syringe and the solution heated overnight at 80°C. After stripping the solvent 50 mL water were added and the solution was extracted with chloroform (5 x 30 mL). The solution was dried over Na₂SO₄ and after stripping the solvent the crude product was purified by column chromatography (CH₂Cl₂/CH₃OH 98/2).

Product characteristics:
Appearance: Yellow oil
Yield: 130 mg (0.46 mmol, 46%)

Thin-layer chromatography (silica gel):
R_{f} 3-Bromo-4-methylpyridin (CH₂Cl₂/CH₃OH 98/2) = 0.50
R_{f} 3-(6-(2-Fluoroethoxy)-2-naphthyl)-4-methylpyridine (CH₂Cl₂/CH₃OH 98/2) = 0.20
¹H-NMR (CDCl₃): δ = 8.50 (m, 2H), 7.85 (d, 2H), 7.75 (s, 1H), 7.40 (d, 1H), 7.20 (m, 3H), 4.95 (t, 1H), 4.75 (t, 1H), 4.45 (t, 1H), 4.30 (t, 1H), 2.35 (s, 3H).

### Preferred compounds

The compounds of formula (I) preferably have the following substituents:
**1.** If R₁ represents CH₂CH₂X then:
   1.1 - R₂ and R₃ are H and R₄ is one of the following substituents a, b, c, d or e: 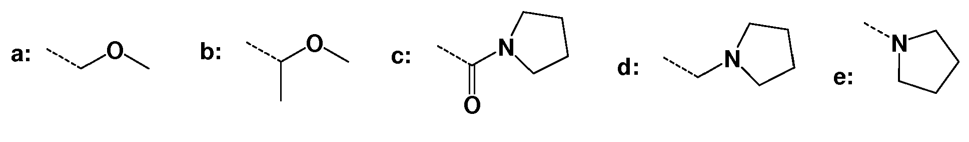
   1.2 - or R₂ and R₄ are H and R₃ is one of the following substituents a, b, c, or d: 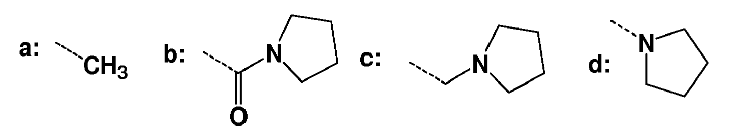
   1.3 - or R₂ is H and R₃ and R₄ forms together with the pyridine ring an isochinoline ring system.
2. If R₁ is CH₃ then:
   2.1 - R₂ and R₃ are H und R₄ is one of the following substituents a, b, c, d or e: 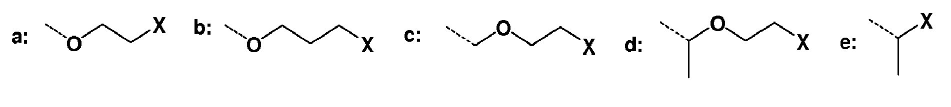
   2.2 - or R₂ is one of the following substituents a or b: 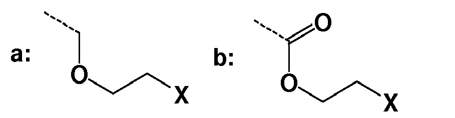 and R₃ and R₄ form together with the pyridine ring an isochinoline ring system.
   2.3 - or R₂ is one of the following substituents a or b: 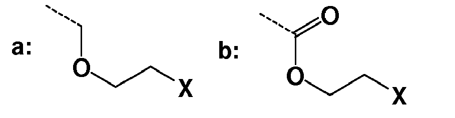 and R₃ is H and R₄ one of the following substituents a or b:

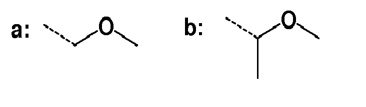

Preferred compounds include:

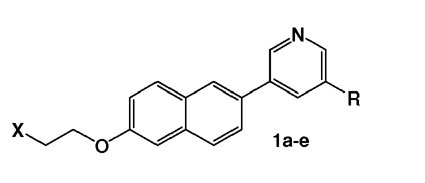

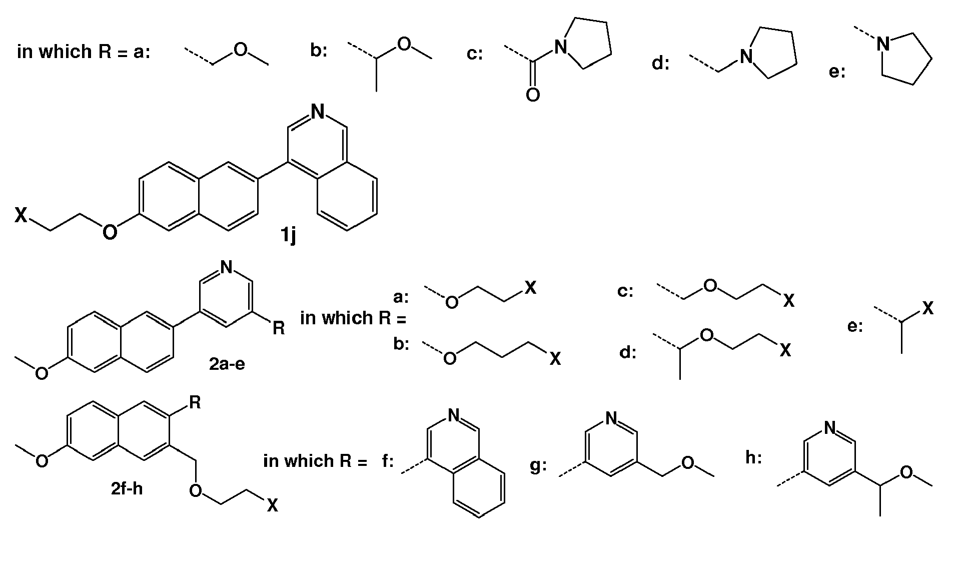

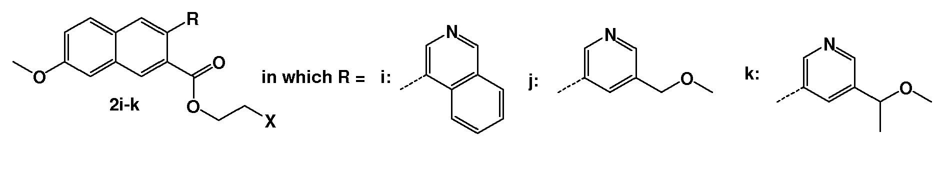

In the above compounds, the variable "X" has the same meaning as defined above. In particular, "X" represents ¹⁸F when the compound is a radioactive tracer and "X" represents a leaving group such as Br, I, tosylate or mesylate when the compound is a precursor for making the radioactive tracer.

Several non-radioactive fluorinated analogs of the above radioactive tracers were prepared for testing affinity for CYP11 B2 (aldosterone synthase), expressed as lC₅₀, and selectivity for CYP11 B2 over CYP11 B1 (11β-hydroxylase), referred to herein as the selectivity factor, *in vitro* as follows:

### Synthesis of Nonradioactive Reference Compound 1a*

### Step 1: Synthesis of 2-fluoroethyl tosylate

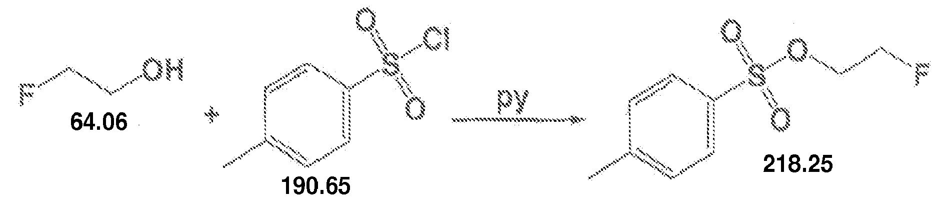

A solution of 22.9 mL (25 g, 391 mmol) 2-fluoroethanol in 300 mL pyridine was cooled in an ice bath, and 163 g (850 mmol) tosyl chloride was added thereto in portions over a period of 30 min, under argon. Stirring was performed for an additional 4 hours on the ice bath, and 300 g ice and 500 mL water were then added. After adding 1000 mL ethyl acetate the phases were separated, and the organic phase was washed with 500 mL of a 5% sodium carbonate solution and 200 mL water. After drying over sodium sulfate and stripping the solvent, the crude product was obtained as an oil, which was purified by distillation in an oil pump vacuum (approximately 100°C at 1-2 mbar).

61.27 g (280.7 mmol, 71.8% yield) of a colorless liquid was obtained.

Product characteristics:
Thin-layer chromatography (silica gel):
R_{f} tosyl chloride (CH₂Cl₂/CH₃OH 98/2) = 0.85
R_{f} 2-fluoroethyl tosylate (CH₂Cl₂/CH₃OH 98/2) = 0.35
¹H-NMR (CDCl₃): δ = 7.81 (d, 2H), 7.38 (d, 2H), 4.65 (t, 1H), 4.53 (t, 1H), 4.32 (t, 1H), 4.25 (t, 1H), 2.48 (s, 3H)

### Step 2: Synthesis of 3-bromo-5-(2-fluoroethoxy)pyridine

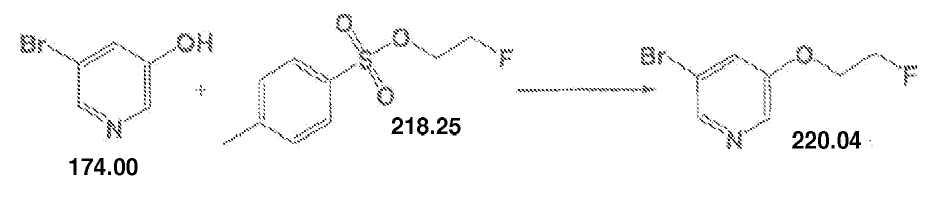

A solution of 1.0 g (5.75 mmol) 3-bromo-5-hydroxypyridine, 1.85 g (13.2 mmol) K₂CO₃, and 2.25 g (10.3 mmol) 2-fluoroethyl tosylate in 14 mL DMF was heated overnight at 60°C. After cooling to room temperature the solvent was stripped on the oil pump, and the residue was taken up in 100 mL water and extracted three times each with 50 mL chloroform. After stripping the solvent the crude product was obtained, which was purified by column chromatography:
1: 500 mL CH₂Cl₂
2: CH₂Cl₂/CH₃OH 98/2
1.04 g (4.73 mmol, 82.2% yield) of a yellow liquid was obtained.

Product characteristics:
Thin-layer chromatography (silica gel):
R_{f} 3-bromo-5-hydroxypyridine (CH₂Cl₂) = 0.05
R_{f} 3-bromo-5-(2-fluoroethoxy)pyridine (CH₂Cl₂) = 0.25
¹H-NMR (CDCl₃): δ = 8.28 (d, 1H), 8.22 (d, 1H), 7.30 (t, 1H), 4.75 (t, 1H), 4.60 (t, 1H), 4.28 (t, 1H), 4.17 (t, 1H)

### Step 3: Synthesis of 3-(6-methoxy-2-naphthyl)-5-(2-fluoroethoxy)pyridine (1a*)

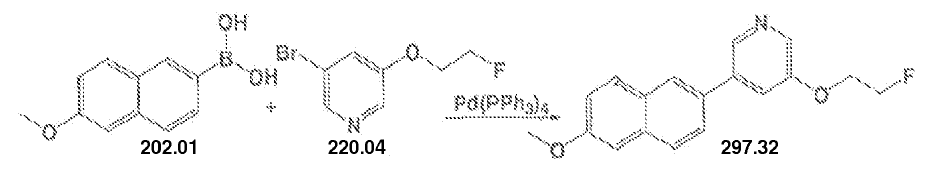

A solution of 642 mg (2.5 mmol) 6-methoxy-2-naphthalene boronic acid, 700 mg (3.18 mmol) 3-bromo-5-(2-fluoroethoxy)pyridine, 2.10 g (19.7 mmol) Na₂CO₃ in 9 mL water, and 137 mg (0.013 mmol) tetrakis(triphenylphosphine)palladium in 31 mL methanol was heated overnight at 80°C, under argon. After stripping the solvent, the residue was dissolved in 50 mL water, extracted three times with dichloromethane, and dried over sodium sulfate, and after stripping the solvent the crude product was purified by column chromatography:
1. CH₂Cl₂ (500 mL)
2. CH₂Cl₂/MeOH 98/2

669 mg (2.25 mmol, 90.0% yield) of a white solid was obtained.

Product characteristics:
Melting point: 139-141 °C

Thin-layer chromatography (silica gel):
R_{f} 3-bromo-5-(2-fluoroethoxy)pyridine (CH₂Cl₂) = 0.25
R_{f} 3-(6-methoxy-2-naphthyl)-5-(2-fluoroethoxy)pyridine (CH₂Cl₂) = 0.10
¹H-NMR (CDCl₃): δ = 8.59 (d, 1H), 8.30 (d, 1H), 7.90 (s, 1H), 7.75 (t, 2H), 7.61 (d, 1H), 7.43 (t, 1H), 7.19 (m, 2H), 4.83 (t, 1H), 4.62 (t, 1H), 4.33 (t, 1H), 4.17 (t, 1H), 3.90 (s, 3H)

Nonradioactive compounds corresponding to 1 b, 1j, 2a, 2c, 2d, and 2e above were prepared by analogy to the above-described procedure for preparing nonradioactive compound 1a*. Those compounds are hereafter designated 1b*, 1j*, 2a*, 2c*, 2d*, and 2e*. They have the same chemical formulae as compounds 1b, 1j, 2a, 2c, 2d, and 2e described above, except that the fluorine atom is not a radioactive isotope.

To conduct *in vitro* testing of nonradioactive compounds 1a*, 1b*, 1j*, 2a*, 2c*, 2d*, and 2e* to determine IC₅₀ values for inhibition of CYP11B1 and CYP11 B2, human CYP11 B1 and CYP11 B2 enzymes were expressed in Y1 cells using liposome/lipid-mediated DNA transfection. To evaluate CYP11 B1 and CYP11B2 inhibition, hsCYP11 B1- and hsCYP11 B2-expressing Y1 cells were subcultured on 6-well plates (0.5 x 10⁶ cells/well) in 2 ml of culture medium. The enzyme reaction was started after 24 hours by the addition of 1 ml culture medium containing either 11-deoxycortisol (RSS) or 11-deoxycorticosterone (DOC) as substrate and the corresponding inhibitor. RSS and DOC were dissolved in ethanol to a final test concentration of 1 µM. For determination of IC₅₀ values, the inhibitors were added to the culture medium at concentrations between 0.1 nM - 10 µM and incubated for 48 hours. Cells which were treated in the same way but without inhibitors, served as controls. As further controls, untransfected Y1 cells were also incubated with RSS and DOC, respectively. Both, RSS and DOC were obtained from Sigma (Deisenhofen, Germany).

The results obtained for the above-defined compounds are presented in the following table.

| **Non-radioactive Compound** | **IC₅₀** **Aldosterone Synthase [nM]** | **Selectivity Factor** **(lC₅₀ CYP11 B1/ IC₅₀ CYP11B2)** |
|---|---|---|
| 1a | 6.5 ± 3.8 | 104 |
| 1b | 5.2 ± 3.2 | 38 |
| 1j | 15.8 ± 5.1 | > 100 |
| 2a | 18.3 ± 8.4 | 70 |
| 2c | 8.5 ± 3.7 | 140 |
| 2d | 6.0 ± 3.1 | 35 |
| 2e | 8.8 ± 3.9 | > 1000 |

As can be seen from the data shown in the above table, compounds corresponding to the radioactive tracers described herein bind selectively with aldosterone synthase, showing that the corresponding radioactive tracers can be used to conduct PET imaging of aldosterone synthase activity within the living body of a mammal having adrenal glands.

Overall, the invention described herein overcomes the following hurdles:
1. The method allows the enzyme density to be quantifiably determined.
2. The resolution of the method is sufficient to detect Conn adenomas.
3. The radioactive tracer has a physical half-life in the range of a few hours to minimize exposure of living tissues to radiation.
4. To minimize undesired side effects, dosage of radioactive tracer required to differentiate between unilateral and bilateral hyperplasia is extremely small (< 1 µg).
5. Since the enzyme density of aldosterone synthase is high only in the defined region of the zona glomerulosa, i.e., the adenoma, the radioactive tracer has a high affinity for the target enzyme, such as an IC₅₀ value of < 25 nM, preferably < 10 nM..
6. The greatest problem is the existence of a second enzyme, 11β-hydroxylase (CYP11 B1). This enzyme has a high degree of homology (95%) to aldosterone synthase, but is not over-expressed in primary hyperaldosteronism and therefore is not a suitable target enzyme. This difficulty is compounded by the fact that in the normal adrenals 11 β-hydroxylase is expressed at a higher level than is aldosterone synthase. Thus, a suitable radioactively labeled enzyme inhibitor should bind selectively only to the aldosterone synthase, but this represents a problem due to the similarity of the two enzymes. The radioactive tracer of the present invention has a selectivity factor for CYP11 B2 versus CYP11 B1 of at least 5, more preferably at least 10.
7. The radioactive tracers of the present invention can be manufactured from a precursor of the present invention within a time period equal to or less than the half-life of ¹⁸F. In a preferred embodiment, that time period is one hour or less.
8. The radioactive tracers of the present invention can be manufactured from a precursor of the present invention in a one-pot reaction, which is preferably automated to reduce the risk of radiation exposure to personnel conducting the reaction.

The PET analysis method of the present invention permits the difficult and clinically important differential diagnosis between unilateral and bilateral forms of primary hyperaldosteronism. The described disadvantages of the adrenal venous catheter may be avoided by using this noninvasive method. The radioactive tracers of the present invention may be efficiently produced, and due to the use of fluorine-18 as a labeling nuclide may also be easily shipped to clinics and private practices which have their own PET device, but no cyclotron or radiochemistry capability.

## Claims

1. A method for making a functional image of adrenal glands comprising (1) introducing a radioactively labelled CYP11 B2 (aldosterone synthase) inhibitor which binds selectively to CYP11 B2 relative to CYP11 B1 (11β-hydroxylase) into a mammal with adrenal glands and (2) conducting positron emission tomography (PET) in the region of the adrenal glands to obtain a functional image of the adrenal glands.

2. The method according to claim 1, wherein the radioactively labelled CYP11 B2 inhibitor comprises ¹⁸F.

3. The method according to claim 1 or 2, wherein the radioactively labelled CYP11 B2 inhibitor is one or more compounds having the formula (I): 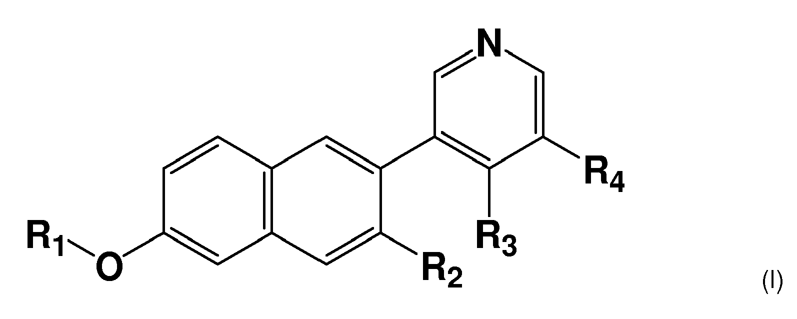 wherein
R₁ represents ―(CH₂)₂-¹⁸F or -CH₃;
R₂ represents ―H, -CH₂O(CH₂)₂-¹⁸F or ―C(O)O(CH₂)₂-¹⁸F;
R₃ represents -H, -CH₃, -C(O)-N-pyrrolidine, -CH₂-N-pyrrolidine, or -N-pyrrolidine;
R₄ represents ―H, -CH₂-O-CH₃, -CH(CH₃)OCH₃, -C(O)-N-pyrrolidine, -CH₂-N-pyrrolidine, ―N-pyrrolidine, -CH(CH₃)-¹⁸F, -O(CH₂)₂-¹⁸F, -O(CH₂)₃-¹⁸F, - CH₂-O-(CH₂)₂-¹⁸F, or ―CH(CH₃)-O-(CH₂)₂-¹⁸F, or
R₁ and R₂ are defined as above and R₃ and R₄ together with the pyridinyl ring of formula (I) form a isochinoline ring system,
wherein one of R₁, R₂ or R₄ represents a group having an ¹⁸F moiety.

4. A compound having the formula (I): 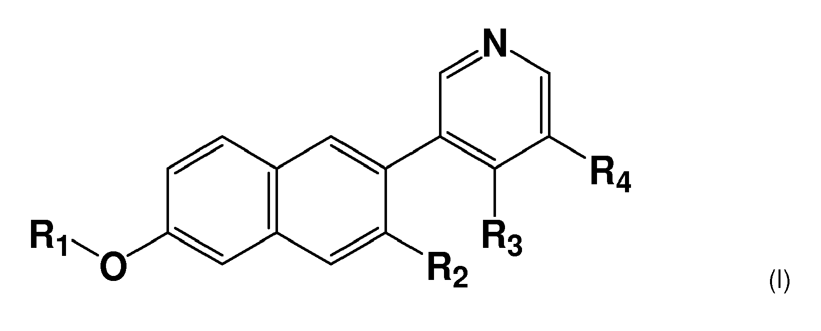 wherein
R₁ represents ―(CH₂)₂-X or -CH₃;
R₂ represents ―H, -CH₂O(CH₂)₂-X or ―C(O)O(CH₂)₂-X;
R₃ represents -H, -CH₃, -C(O)-N-pyrrolidine, -CH₂-N-pyrrolidine, or -N-pyrrolidine;
R₄ represents ―H, -CH₂-O-CH₃, -CH(CH₃)OCH₃, -C(O)-N-pyrrolidine, -CH₂-N-pyrrolidine, -N-pyrrolidine, -CH(CH₃)-X, -O(CH₂)₂-X, -O(CH₂)₃-X, -CH₂-O-(CH₂)₂-X, or ―CH(CH₃)-O-(CH₂)₂-X, or
R₁ and R₂ are defined as above and R₃ and R₄ together with the pyridinyl ring of formula (I) form an isochinoline ring system,
wherein one of R₁, R₂ or R₄ represents a group having an X moiety and X represents ¹⁸F, Br, I, tosylate or mesylate.

5. The compound of claim 4, wherein R₁ represents CH₂CH₂X, R₂ and R₃ are H and R₄ is one of the following substituents a, b, c, d or e:

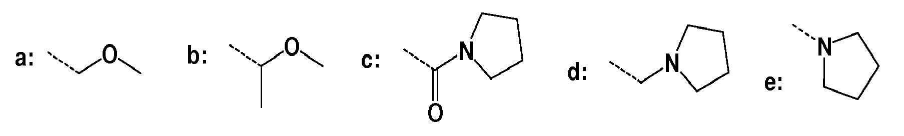

6. The compound of claim 4, wherein R₁ represents CH₂CH₂X, R₂ and R₄ are H and R₃ is one of the following substituents a, b, c, d:

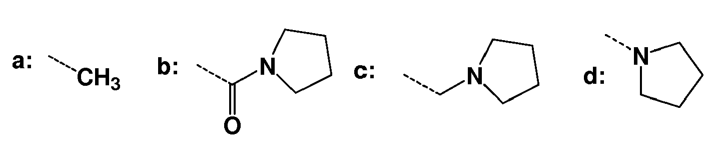

7. The compound of claim 4, wherein R₁ represents CH₂CH₂X, R₂ is H and R₃ and R₄ form, together with the pyridine ring, an isochinoline ring system.

8. The compound of claim 4, wherein R₁ is CH₃, R₂ and R₃ are H und R₄ is one of the following substituents a, b, c, d, or e:

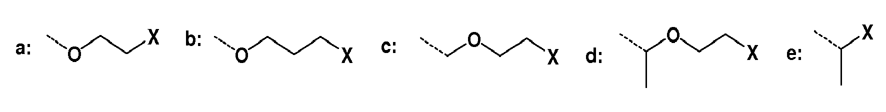

9. The compound of claim 4, wherein R₁ is CH₃, R₂ is one of the following substituents: 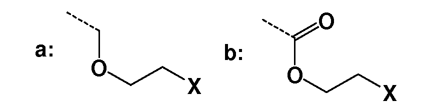 and R₃ and R₄ form, together with the pyridine ring, an isochinoline ring system.

10. The compound of claim 4, wherein R₁ is CH₃, R₂ is one of the following substituents a or b: 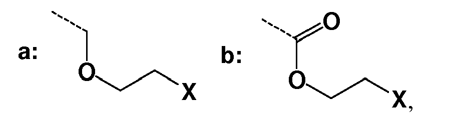 R₃ is H, and R₄ is one of the following substituents a or b:

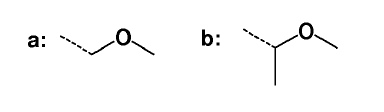

11. The compound of claim 4 represented by the formula: 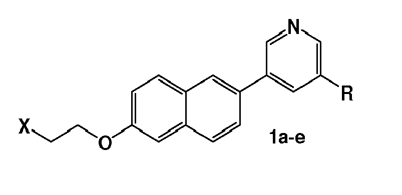 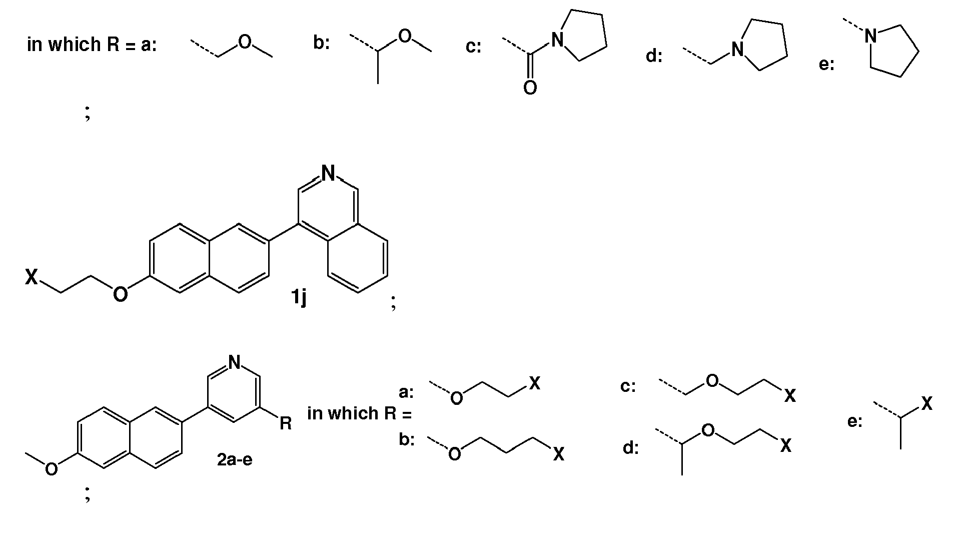 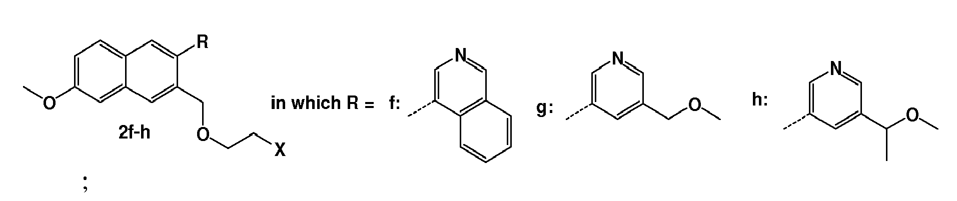 or

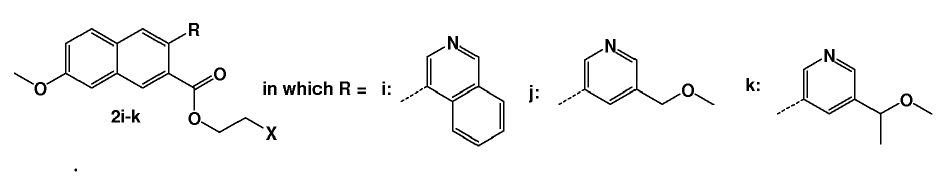

12. A process for making a radioactive tracer comprising reacting a compound of formula (I) according to any one of claims 4 to 11, wherein X represents Br, I, tosylate or mesylate, with ¹⁸F ions to obtain a radioactive tracer compound of formula (I) in which X represents ¹⁸F.

13. The process of claim 12, wherein the process is conducted in the presence of a cyclic crown ether compound, a potassium salt, and an anhydrous polar aprotic organic solvent.

14. The process of claim 12 or 13, wherein the process is conducted at a temperature in the range from 70°C to 100°C for a time period of less than 20 minutes.
